# EUROPÄISCHE PATENTANMELDUNG

(11) **EP 1 096 013 A2**
(43) Veröffentlichungstag der Anmeldung: **02.05.2001**
(21) Anmeldenummer: 00122505.1
(22) Anmeldetag: 14.10.2000
(51) Int. Cl.: C12N 15/53, C12N 9/02, C12P 13/08

(54) **Corynebacterium poxB-Gen codierende Nukleotidsequenzen, und die Verwendung zur Herstellung von L-Lysin**

(30) Priorität: 28.10.1999 DE 19951975
(71) Anmelder: Degussa AG, 40474 Düsseldorf (DE)
(72) Erfinder: Möckel, Bettina, Dr., 40597 Düsseldorf (DE); Weissenborn, Anke, 72076 Tübingen (DE); Pfefferle, Walter, Dr., 33790 Halle (Westf.) (DE); Pühler, Alfred, Prof., 33739 Bielefeld (DE); Kalinowski, Jörn, Dr., 33615 Bielefeld (DE); Bathe, Brigitte, Dr., 33154 Salzkotten (DE); Dusch, Nicole, Dr., 33619 Bielefeld (DE)

(57) **Zusammenfassung**

Isoliertes Polynukleotid enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukleotid, das für ein Polypeptid codiert, das eine Aminosäuresequenz enthält, die zu mindestens 70 % identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Basen der Polynukleotidsequenz von a), b) oder c),
und Verfahren zur fermentativen Herstellung von L-Aminosäuren durch Abschwächung des poxB-Gens.

## Beschreibung

Gegenstand der Erfindung sind für das poxB-Gen kodierende Nukleotidsequenzen aus coryneformen Bakterien und ein Verfahren zur fermentativen Herstellung von Aminosäuren, insbesondere L-Lysin durch Abschwächung des poxB-Gens.

### Stand der Technik

L-Aminosäuren, insbesondere Lysin finden in der Humanmedizin und in der pharmazeutischen Industrie, in der Lebensmittelindustrie und ganz besonders in der Tierernährung Anwendung.

Es ist bekannt, daß Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensbesserungen können fermentationstechnische Maßnahmen wie z.B. Rührung und Versorgung mit Sauerstoff, oder die Zusammensetzung der Nährmedien wie z.B. die Zuckerkonzentration während der Fermentation, oder die Aufarbeitung zur Produktform durch z.B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und Aminosäuren produzieren.

Seit einigen Jahren werden ebenfalls Methoden der rekombinanten DNA-Technik zur Stammverbesserung von L-Aminosäure produzierenden Stämmen von Corynebacterium eingesetzt.

### Aufgabe der Erfindung

Die Erfinder haben sich zur Aufgabe gestellt, neue Maßnahmen zur verbesserten fermentativen Herstellung von Aminosäuren insbesondere L-Lysin bereitzustellen.

### Beschreibung der Erfindung

L-Aminosäuren, insbesondere Lysin finden in der Humanmedizin und in der pharmazeutischen Industrie, in der Lebensmittelindustrie und ganz besonders in der Tierernährung Anwendung. Es besteht daher ein allgemeines Interesse daran, neue verbesserte Verfahren zur Herstellung von Aminosäuren, insbesondere L-Lysin, bereitzustellen.

Gegenstand der Erfindung ist ein isoliertes Polynukleotid enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukleotid, das für ein Polypeptid codiert, das eine Aminosäuresequenz enthält, die zu mindestens 70% identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Basen der Polynukleotidsequenz von a), b) oder c).

Gegenstand der Erfindung ist ebenfalls das Polynukleotid gemäß Anspruch 1, wobei es sich bevorzugt um eine replizierbare DNA handelt, enthaltend:
(i) die Nukleotidsequenz, gezeigt in SEQ ID No. 1, oder
(ii) mindestens eine Sequenz, die der Sequenz (i) innerhalb des Bereichs der Degeneration des genetischen Codes entspricht, oder
(iii) mindestens eine Sequenz, die mit der zur Sequenz (i) oder (ii) komplementären Sequenz hybridisiert, und gegebenenfalls
(iv) funktionsneutralen Sinnmutationen in (i).

Weitere Gegenstände sind
ein Polynukleotid gemäß Anspruch 2, enthaltend die Nukleotidsequenz wie in SEQ ID No. 1 dargestellt,
ein Polynukleotid gemäß Anspruch 2, das für ein Polypeptid codiert, das die Aminosäuresequenz, wie in SEQ ID No. 2 dargestellt, enthält
ein Vektor, enthaltend das Polynukleotid gemäß Anspruch 1, Punkt d insbesondere pCR2.1poxBint, hinterlegt in E.coli DSM 13114
und als Wirtszelle dienende coryneforme Bakterien, die in dem pox-Gen eine Insertion oder Delektion enthalten.

Gegenstand der Erfindung sind ebenso Polynukleotide, die im wesentlichen aus einer Polynukleotidsequenz bestehen, die erhältlich sind durch Screening mittels Hybridisierung einer entsprechenden Genbank, die das vollständige Gen mit der Polynukleotidsequenz entsprechend SEQ ID No. 1 enthalten mit einer Sonde, die die Sequenz des genannten Polynukleotids gemäß SEQ ID No. 1 oder ein Fragment davon enthält und Isolierung der genannten DNA-Sequenz.

Polynukleotidsequenzen gemäß der Erfindung sind geeignet als Hybridisierungs-Sonden für RNA, cDNA und DNA, um cDNA in voller Länge zu isolieren, die für Pyruvat-Oxidase codieren und solche cDNA oder Gene zu isolieren, die eine hohe Ähnlichkeit der Sequenz mit der des Pyruvat-Oxidase Gens aufweisen.

Polynukleotidsequenzen gemäß der Erfindung sind weiterhin als Primer geeignet, mit deren Hilfe mit der Polymerase Kettenreaktion (PCR) DNA von Genen hergestellt werden kann, die für Pyruvat-Oxidase codieren.

Solche als Sonden oder Primer dienende Oligonukleotide enthalten mindestens 30, bevorzugt mindestens 20, ganz besonders bevorzugt mindestens 15 aufeinanderfolgende Nukleotide. Geeignet sind ebenfalls Oligonukleotide mit einer Länge von mindestens 40 oder 50 Basen.

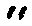 Isoliert" bedeutet aus seinem natürlichen Umfeld herausgetrennt.

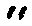 Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Unter 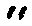 Polypeptiden" versteht man Peptide oder Proteine, die zwei oder mehr über Peptidbindungen verbundene Aminosäuren enthalten.

Die Polypeptide gemäß Erfindung schließen das Polypeptid gemäß SEQ ID No. 2, insbesondere solche mit der biologischen Aktivität der Pyruvat-Oxidase und auch solche ein, die zu wenigstens 70% identisch sind mit dem Polypeptid gemäß SEQ ID No. 2, bevorzugt zu wenigstens 80% und besonders zu wenigstens 90 % bis 95 % Identität mit dem Polypeptid gemäß SEQ ID No. 2 und die genannte Aktivität aufweisen.

Die Erfindung betrifft weiterhin ein Verfahren zur fermentativen Herstellung von Aminosäuren, insbesondere Lysin, unter Verwendung von coryneformen Bakterien, die insbesondere bereits die Aminosäuren, insbesondere L-Lysin produzieren und in denen die für das poxB-Gen codierenden Nukleotidsequenzen abgeschwächt, insbesondere auf niedrigem Niveau exprimiert werden.

Der Begriff 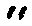 Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym (Protein) inaktiviert und gegebenenfalls diese Maßnahmen kombiniert.

Die Mikroorganismen, die Gegenstand der vorliegenden Erfindung sind, können Aminosäuren, insbesondere Lysin aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind besonders die bekannten Wildtypstämme
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte L-Aminosäuren produzierende Mutanten bzw. Stämme, wie beispielsweise die L-Lysin produzierenden Stämme
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 und
Corynebacterium glutamicum DSM 5714 Den Erfindern
gelang es, das neue, für das Enzym Pyruvat-Oxidase (EC 1.2.2.2) kodierende poxB-Gen von C. glutamicum zu isolieren.

Zur Isolierung des poxB-Gens oder auch anderer Gene von C. glutamicum wird zunächst eine Genbank dieses Mikroorganismus in E. coli angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495 - 508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E. coli K-12 Stamm NM554 (Raleigh et al., 1988, Nucleic Acids Research 16:1563-1575) angelegt wurde. Börmann et al. (Molecular Microbiology 6(3), 317-326, 1992) wiederum beschreiben eine Genbank von C. glutamicum ATCC13032 unter Verwendung des Cosmids pHC79 (Hohn und Collins, Gene 11, 291-298 (1980)). O'Donohue (The Cloning and Molecular Analysis of Four Common Aromatic Amino Acid Biosynthetic Genes from Corynebacterium glutamicum. Ph.D. Thesis, National University of Ireland, Galway, 1997) beschreibt die Klonierung von C. glutamicum Genen unter Verwendung des von Short et al. (Nucleic Acids Research, 16: 7583) beschriebenen λ Zap Expressionssystems.

Zur Herstellung einer Genbank von C. glutamicum in E. coli können auch Plasmide wie pBR322 (Bolivar, Life Sciences, 25, 807-818 (1979)) oder pUC9 (Vieira et al., 1982, Gene, 19:259-268) verwendet werden. Als Wirte eignen sich besonders solche E. coli-Stämme, die restriktions- und rekombinationsdefekt sind wie beispielsweise der Stamm DH5α (Jeffrey H. Miller: 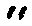 A Short Course in Bacterial Genetics, A Laboratory Manual and Handbook for Escherichia coli and Related Bacteria", Cold Spring Harbour Laboratory Press, 1992).

Die mit Hilfe von Cosmiden oder anderen λ-Vektoren klonierten langen DNA-Fragmente können anschließend wiederum in gängige für die DNA-Sequenzierung geeignete Vektoren subkloniert werden.

Methoden zur DNA-Sequenzierung sind unter anderem bei Sanger et al. (Proceedings of the National Academy of Sciences of the United States of America USA, 74:5463-5467, 1977) beschrieben.

Die erhaltenen DNA-Sequenzen können dann mit bekannten Algorithmen bzw. Sequenzanalyse-Programmen wie z. B. dem von Staden (Nucleic Acids Research 14, 217-232(1986)),dem GCG-Programm von Butler (Methods of Biochemical Analysis 39, 74-97 (1998)) dem FASTA-Algorithmus von Pearson und Lipman (Proceedings of the National Academy of Sciences USA 85,2444-2448 (1988)) oder dem BLAST-Algorithmus von Altschul et al. (Nature Genetics 6, 119-129 (1994)) untersucht und mit den in öffentlich zugänglichen Datenbanken vorhandenen Sequenzeinträgen verglichen werden. Öffentlich zugängliche Datenbanken für Nukleotidsequenzen sind beispielsweise die der European Molecular Biologies Laboratories (EMBL, Heidelberg, Deutschland) oder die des National Center for Biotechnology Information (NCBI, Bethesda, MD, USA).

Auf diese Weise wurde die neue für das poxB-Gen kodierende DNA-Sequenz von C. glutamicum erhalten, die als SEQ ID No. 1 Bestandteil der vorliegenden Erfindung ist. Weiterhin wurde aus der vorliegenden DNA-Sequenz mit den oben beschriebenen Methoden die Aminosäuresequenz des entsprechenden Proteins abgeleitet. In SEQ ID No. 2 ist die sich ergebende Aminosäuresequenz des poxB-Genproduktes dargestellt.

Kodierende DNA-Sequenzen, die sich aus SEQ ID No. 1 durch die Degeneriertheit des genetischen Codes ergeben, sind ebenfalls Bestandteil der Erfindung. In gleicher Weise sind DNA-Sequenzen, die mit SEQ ID No. 1 oder Teilen von SEQ ID No. 1 hybridisieren Bestandteil der Erfindung. Schließlich sind DNA-Sequenzen Bestandteil der Erfindung, die durch die Polymerase-Kettenreaktion (PCR) unter Verwendung von Primern hergestellt werden, die sich aus SEQ ID No. 1 ergeben.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260). Anleitungen zur Amplifikation von DNA-Sequenzen mit Hilfe der Polymerase-Kettenreaktion (PCR) findet der Fachmann unter anderem im Handbuch von Gait: Oligonukleotide synthesis: a practical approach (IRL Press, Oxford, UK, 1984) und bei Newton und Graham: PCR (Spektrum Akademischer Verlag, Heidelberg, Deutschland, 1994).

Die Erfinder fanden heraus, daß coryneforme Bakterien nach Abschwächung des poxB-Gens in verbesserter Weise L-Aminosäuren insbesondere L-Lysin produzieren.

Zur Erzielung einer Abschwächung können entweder die Expression des poxB-Gens oder die katalytischen Eigenschaften des Enzymproteins herabgesetzt oder ausgeschaltet werden. Gegebenenfalls können beide Maßnahmen kombiniert werden.

Die Erniedrigung der Genexpression kann durch geeignete Kulturführung oder durch genetische Veränderung (Mutation) der Signalstrukturen der Genexpression erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann z. B. in der Patentanmeldung WO 96/15246, bei Boyd und Murphy (Journal of Bacteriology 170: 5949 (1988)), bei Voskuil und Chambliss (Nucleic Acids Research 26: 3548 (1998), bei Jensen und Hammer (Biotechnology and Bioengineering 58: 191 (1998)), bei Patek et al. (Microbiology 142: 1297 (1996) und in bekannten Lehrbüchern der Genetik und Molekularbiologie wie z. B. dem Lehrbuch von Knippers ( 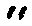 Molekulare Genetik", 6. Auflage, Georg Thieme Verlag, Stuttgart, Deutschland, 1995) oder dem von Winnacker ( 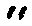 Gene und Klone", VCH Verlagsgesellschaft, Weinheim, Deutschland, 1990).

Mutationen, die zu einer Veränderung bzw. Herabsetzung der katalytischen Eigenschaften von Enzymproteinen führen sind aus dem Stand der Technik bekannt; als Beispiele seien die Arbeiten von Qiu und Goodman (Journal of Biological Chemistry 272: 8611-8617 (1997)), Sugimoto et al. (Bioscience Biotechnology and Biochemistry 61: 1760-1762 (1997)) und Möckel ( 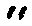 Die Threonindehydratase aus Corynebacterium glutamicum: Aufhebung der allosterischen Regulation und Struktur des Enzyms", Berichte des Forschungszentrums Jülichs,Jül-2906, ISSN09442952, Jülich, Deutschland, 1994) genannt. Zusammenfassende Darstellungen können bekannten Lehrbüchern der Genetik und Molekularbiologie wie z. B. dem von Hagemann ( 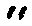 Allgemeine Genetik", Gustav Fischer Verlag, Stuttgart, 1986) entnommen werden.

Als Mutationen kommen Transitionen, Transversionen, Insertionen und Deletionen in Betracht. In Abhängigkeit von der Wirkung des Aminosäureaustausches auf die Enzymaktivität wird von Fehlsinnmutationen (missense mutations) oder Nichtsinnmutationen (nonsense mutations) gesprochen. Insertionen oder Deletionen von mindestens einem Basenpaar in einem Gen führen zu Rasterverschiebungsmutationen (frame shift mutations) in deren Folge falsche Aminosäuren eingebaut werden oder die Translation vorzeitig abbricht. Deletionen von mehreren Kodonen führen typischerweise zu einem vollständigen Ausfall der Enzymaktivität. Anleitungen zur Erzeugung derartiger Mutationen gehören zum Stand der Technik und können bekannten Lehrbüchern der Genetik und Molekularbiologie wie z. B. dem Lehrbuch von Knippers ( 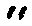 Molekulare Genetik", 6. Auflage, Georg Thieme Verlag, Stuttgart, Deutschland, 1995), dem von Winnacker ( 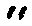 Gene und Klone", VCH Verlagsgesellschaft, Weinheim, Deutschland, 1990)oder dem von Hagemann ( 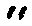 Allgemeine Genetik", Gustav Fischer Verlag, Stuttgart, 1986) entnommen werden.

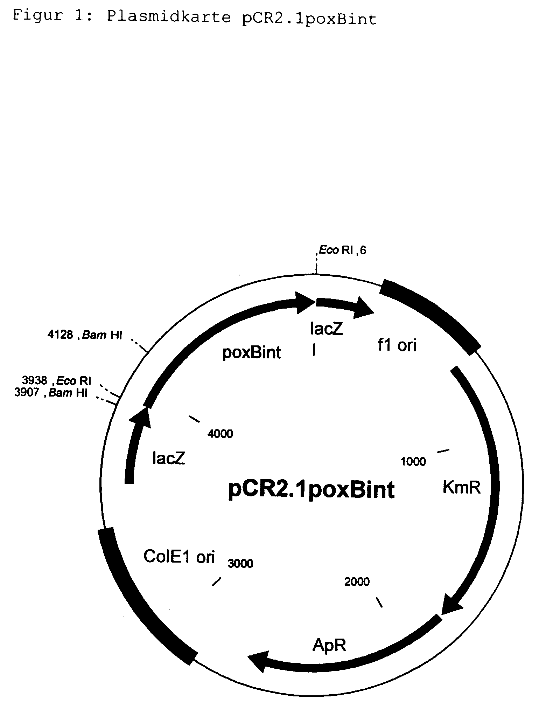

Ein Beispiel für ein Plasmid, mit Hilfe dessen eine Insertionsmutagenese des poxB-Gens durchgeführt werden kann, ist pCR2.1poxBint (Figur 1).

Plasmid pCR2.1poxBint besteht aus dem von Mead at al. (Bio/Technology 9:657-663 (1991)) beschriebenen Plasmid pCR2.1-TOPO, in das ein internes Fragment des poxB-Gens, dargestellt in SEQ-ID No. 3, eingebaut wurde. Dieses Plasmid führt nach Transformation und homologer Rekombination in das chromosomale poxB-Gen (Insertion) zu einem Totalverlust der Enzymfunktion. Auf diese Weise wurde beispielhaft der Stamm DSM5715::pCR2.1poxBint hergestellt, dessen Pyruvat-Oxidase ausgeschaltet ist. Weitere Anleitungen und Erläuterungen zur Insertionsmutagenese findet man beispielsweise bei Schwarzer und Pühler (Bio/Technology 9,84-87 (1991)) oder Fitzpatrick et al. (Applied Microbiology and Biotechnology 42, 575-580 (1994)).

Zusätzlich kann es für die Produktion von L-Aminosäuren insbesondere L-Lysin vorteilhaft sein, zusätzlich zur Abschwächung des poxB-Gens eines oder mehrere Enzyme des jeweiligen Biosyntheseweges, der Glykolyse, der Anaplerotik, des Zitronensäure-Zyklus oder des Aminosäure-Exports zu verstärken, insbesondere zu überexprimieren.

So kann beispielsweise für die Herstellung von L-Lysin
- gleichzeitig das für die Dihydrodipicolinat-Synthase kodierende dapA-Gen (EP-B 0 197 335), oder
- gleichzeitig das für die Tetradihydrodipicolinat Succinylase kodierende dapD Gen (Wehrmann et al., Journal of Bacteriology 180, 3159-3165 (1998)), oder
- gleichzeitig das Gen für die Succinyldiaminopimelate-Desuccinylase kodierende dapE Gen (Wehrmann et al., Journal of Bacteriology 177: 5991-5993 (1995)), oder
- gleichzeitig das für die Glyceraldehyd-3-Phosphat Dehydrogenase kodierende gap-Gen (Eikmanns (1992). Journal of Bacteriology 174:6076-6086), oder
- gleichzeitig das für die Pyruvat Carboxylase codierende pyc-Gen(Eikmanns (1992). Journal of Bacteriology 174:6076-6086), oder
- gleichzeitig das für die Malat:Chinon Oxidoreduktase kodierende mqo-Gen (Molenaar et al., European Journal of Biochemistry 254, 395 - 403 (1998)), oder
- gleichzeitig das für den Lysin-Export kodierende lysE-Gen (DE-A-195 48 222)

### überexprimiert werden.

Weiterhin kann es für die Produktion von Aminosäuren, insbesondere L-Lysin vorteilhaft sein, neben der Abschwächung des poxB-Gens unerwünschte Nebenreaktionen auszuschalten (Nakayama: 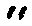 Breeding of Amino Acid Producing Micro-organisms", in: Overproduction of Microbial Products, Krumphanzl, Sikyta, Vanek (eds.), Academic Press, London, UK, 1982).

Die das Polynukleotid gemäß Anspruch 1 enthaltenden Mikroorganismen sind ebenfalls Gegenstand der Erfindung und können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren insbesondere L-Lysin kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedener Mikroorganismen sind im Handbuch 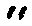 Manual of Methods for General Bacteriology 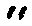 der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoffhaltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten werden Sauerstoff oder Sauerstoff haltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt bis sich ein Maximum des gewünschten Produktes gebildet hat. Dieses Ziel wird normalerweise innerhalb von 10 Stunden bis 160 Stunden erreicht.

Methoden zur Bestimmung von L-Aminosäuren sind aus dem Stand der Technik bekannt. Die Analyse kann so wie bei Spackman et al. (Analytical Chemistry, 30, (1958), 1190) beschrieben durch Anionenaustauschchromatographie mit anschließender Ninhydrin Derivatisierung erfolgen, oder sie kann durch reversed phase HPLC erfolgen, so wie bei Lindroth et al. (Analytical Chemistry (1979) 51: 1167-1174) beschrieben.

Folgender Mikroorganismus wurde bei der Deutschen Sammlung für Mikrorganismen und Zellkulturen (DSMZ, Braunschweig, Deutschland) gemäß Budapester Vertrag hinterlegt:
- Escherichia coli Stamm DH5α/pCR2.1poxBint als DSM 13114.

### Beispiele

Die vorliegende Erfindung wird im folgenden anhand von Ausführungsbeispielen näher erläutert.

### Beispiel 1

### Herstellung einer genomischen Cosmid-Genbank aus Corynebacterium glutamicum ATCC 13032

Chromosomale DNA aus Corynebacterium glutamicum ATCC 13032 wurde wie bei Tauch et al., (1995, Plasmid 33:168-179) beschrieben, isoliert und mit dem Restriktionsenzym Sau3AI (Amersham Pharmacia, Freiburg, Deutschland, Produktbeschreibung Sau3AI, Code no. 27-0913-02) partiell gespalten. Die DNA-Fragmente wurden mit shrimp alkalischer Phosphatase (Rache Molecular Biochemicals, Mannheim, Deutschland, Produktbeschreibung SAP, Code no. 1758250) dephosphoryliert. Die DNA des Cosmid-Vektors SuperCos1 (Wahl et al. (1987) Proceedings of the National Academy of Sciences USA 84:2160-2164), bezogen von der Firma Stratagene (La Jolla, USA, Produktbeschreibung SuperCos1 Cosmid Vektor Kit, Code no. 251301) wurde mit dem Restriktionsenzym XbaI (Amersham Pharmacia, Freiburg, Deutschland, Produktbeschreibung XbaI, Code no. 27-0948-02) gespalten und ebenfalls mit shrimp alkalischer Phosphatase dephosphoryliert. Anschließend wurde die Cosmid-DNA mit dem Restriktionsenzym BamHI (Amersham Pharmacia, Freiburg, Deutschland, Produktbeschreibung BamHI, Code no. 27-0868-04) gespalten. Die auf diese Weise behandelte Cosmid-DNA wurde mit der behandelten ATCC13032-DNA gemischt und der Ansatz mit T4-DNA-Ligase (Amersham Pharmacia, Freiburg, Deutschland, Produktbeschreibung T4-DNA-Ligase, Code no.27-0870-04) behandelt. Das Ligationsgemisch wurde anschließend mit Hilfe des Gigapack II XL Packing Extracts (Stratagene, La Jolla, USA, Produktbeschreibung Gigapack II XL Packing Extract, Code no. 200217) in Phagen verpackt. Zur Infektion des E. coli Stammes NM554 (Raleigh et al. 1988, Nucleic Acid Res. 16:1563-1575) wurden die Zellen in 10 mM MgSO₄ aufgenommen und mit einem Aliquot der Phagensuspension vermischt. Infektion und Titerung der Cosmidbank wurden wie bei Sambrook et al. (1989, Molecular Cloning: A laboratory Manual, Cold Spring Harbor) beschrieben durchgeführt, wobei die Zellen auf LB-Agar (Lennox, 1955, Virology, 1:190) + 100 µg/ml Ampicillin ausplattiert wurden. Nach Inkubation über Nacht bei 37°C wurden rekombinante Einzelklone selektioniert.

### Beispiel 2

### Isolierung und Sequenzierung des poxB-Gens

Die Cosmid-DNA einer Einzelkolonie wurde mit dem Qiaprep Spin Miniprep Kit (Product No. 27106, Qiagen, Hilden, Germany) nach Herstellerangaben isoliert und mit dem Restriktionsenzym Sau3AI (Amersham Pharmacia, Freiburg, Deutschland, Produktbeschreibung Sau3AI, Product No. 27-0913-02) partiell gespalten. Die DNA-Fragmente wurden mit shrimp alkalischer Phosphatase (Roche Molecular Biochemicals, Mannheim, Deutschland, Produktbeschreibung SAP, Product No. 1758250) dephosphoryliert. Nach gelelektrophoretischer Auftrennung erfolgte die Isolierung der Cosmidfragmente im Größenbereich von 1500 bis 2000 bp mit dem QiaExII Gel Extraction Kit (Product No. 20021, Qiagen, Hilden, Germany). Die DNA des Sequenziervektors pZero-1 bezogen von der Firma Invitrogen (Groningen, Niederlande, Produktbeschreibung Zero Background Cloning Kit, Product No. K2500-01) wurde mit dem Restriktionsenzym BamHI (Amersham Pharmacia, Freiburg, Deutschland, Produktbeschreibung BamHI, Product No. 27-0868-04) gespalten. Die Ligation der Cosmidfragmente in den Sequenziervektor pZero-1 wurde wie von Sambrook et al. (1989, Molecular Cloning: A laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DH5αMCR (Grant, 1990, Proceedings of the National Academy of Sciences U.S.A., 87:4645-4649) elektroporiert (Tauch et al. 1994, FEMS Microbiol Letters, 123:343-7) und auf LB-Agar (Lennox, 1955, Virology, 1:190) mit 50 µg/ml Zeocin ausplattiert. Die Plasmidpräparation der rekombinanten Klone erfolgte mit dem Biorobot 9600 (Product No. 900200, Qiagen, Hilden, Deutschland). Die Sequenzierung erfolgte nach der Dideoxy-Kettenabbruch-Methode von Sanger et al. (1977, Proceedings of the National Academies of Sciences U.S.A., 74:5463-5467) mit Modifikationen nach Zimmermann et al. (1990, Nucleic Acids Research, 18:1067). Es wurde der "RR dRhodamin Terminator Cycle Sequencing Kit" von PE Applied Biosystems(Product No. 403044, Weiterstadt, Deutschland) verwendet. Die gelelektrophoretische Auftrennung und Analyse der Sequenzierreaktion erfolgte in einem "Rotiphorese NF Acrylamid/Bisacrylamid" Gel (29:1) (Product No. A124.1, Roth, Karlsruhe, Germany) mit dem "ABI Prism 377" Sequenziergerät von PE Applied Biosystems (Weiterstadt, Deutschland).

Die erhaltenen Roh-Sequenzdaten wurden anschließend unter Anwendung des Staden-Programpakets (1986, Nucleic Acids Research, 14:217-231) Version 97-0 prozessiert. Die Einzelsequenzen der pZero1-Derivate wurden zu einem zusammenhängenden Contig assembliert. Die computergestützte Kodierbereichsanalyse wurden mit dem Programm XNIP (Staden, 1986, Nucleic Acids Research, 14:217-231) angefertigt. Weitere Analysen wurden mit den "BLAST search programs" (Altschul et al., 1997, Nucleic Acids Research, 25:3389-3402), gegen die non-redundant Datenbank des "National Center for Biotechnology Information" (NCBI, Bethesda, MD, USA) durchgeführt.

Die erhaltene Nukleotidsequenz ist in SEQ ID No. 1 dargestellt. Die Analyse der Nukleotidsequenz ergab ein offenes Leseraster von 1737 Basenpaaren, welches als poxB-Gen bezeichnet wurde. Das poxB-Gen kodiert für ein Polypeptid von 579 Aminosäuren.

### Beispiel 3

### Herstellung eines Integrationsvektors für die Integrationsmutagenese des poxB-Gens

Aus dem Stamm ATCC 13032 wurde nach der Methode von Eikmanns et al. (Microbiology 140: 1817 - 1828 (1994)) chromosomale DNA isoliert. Aufgrund der aus Beispiel 2 für C. glutamicum bekannten Sequenz des poxB-Gens wurden die folgenden Oligonukleotide für die Polymerase Kettenreaktion ausgewählt:
poxBint1:
   5' TGC GAG ATG GTG AAT GGT GG 3'
poxBint2:
   5' GCA TGA GGC AAC GCA TTA GC 3'

Die dargestellten Primer wurden von der Firma MWG Biotech (Ebersberg, Deutschland) synthetisiert und nach der Standard-PCR-Methode von Innis et al. (PCR protocols. A guide to methods and applications, 1990, Academic Press) mit Pwo-Polymerase der Firma Boehringer die PCR Reaktion durchgeführt. Mit Hilfe der Polymerase-Kettenreaktion wurde ein ca. 0,9 kb großen DNA-Fragment isoliert, welches ein internes Fragment des poxB-Gens trägt und in der SEQ ID No. 3 dargestellt ist.

Das amplifizierte DNA Fragment wurde mit dem TOPO TA Cloning Kit der Firma Invitrogen Corporation (Carlsbad, CA, USA; Katalog Nummer K4500-01) in den Vektor pCR2.1-TOPO (Mead at al. (1991) Bio/Technology 9:657-663) ligiert.

Anschließend wurde der E. coli Stamm DH5α mit dem Ligationsansatz (Hanahan, In: DNA cloning. A practical approach. Vol.I. IRL-Press, Oxford, Washington DC, USA, 1985) elektroporiert. Die Selektion von Plasmid-tragenden Zellen erfolgte durch Ausplattieren des Transformationsansatzes auf LB Agar (Sambrook et al., Molecular cloning: a laboratory manual. 2^{nd} Ed. Cold Spring Harbor Laboratory Press, Cold Spring Harbor, N.Y., 1989), der mit 25 mg/l Kanamycin supplementiert worden war. Plasmid-DNA wurde aus einer Transformante mit Hilfe des QIAprep Spin Miniprep Kit der Firma Qiagen isoliert und durch Restriktion mit dem Restriktionsenzym EcoRI und anschließender Agarosegel-Elektrophorese (0,8%) überprüft. Das Plasmid wurde pCR2.1poxBint genannt.

### Beispiel 4

### Integrationsmutagenese des poxB-Gens in dem Lysinproduzenten DSM 5715

Der in Beispiel 2 genannte Vektor pCR2.1poxBint wurde nach der Elektroporationsmethode von Tauch et.al. (FEMS Microbiological Letters, 123:343-347 (1994)) in Corynebacterium glutamicum DSM 5715 elektroporiert. Bei dem Stamm DSM 5715 handelt es sich um einen AEC resistenten Lysin-Produzenten. Der Vektor pCR2.1poxBint kann in DSM5715 nicht selbständig replizieren und bleibt nur dann in der Zelle erhalten, wenn er ins Chromosom von DSM 5715 integriert hat. Die Selektion von Klonen mit ins Chromosom integriertem pCR2.1poxBint erfolgte durch Ausplattieren des Elektroporationsansatzes auf LB Agar (Sambrook et al., Molecular cloning: a laboratory manual. 2^{nd} Ed. Cold Spring Harbor Laboratory Press, Cold Spring Harbor, N.Y.), der mit 15 mg/l Kanamycin supplementiert worden war. Für den Nachweis der Integration wurde das poxBint Fragment nach der Methode "The DIG System Users Guide for Filter Hybridization" der Firma Boehringer Mannheim GmbH (Mannheim, Deutschland, 1993) mit dem Dig-Hybridisierungskit der Firma Boehringer markiert. Chromosomale DNA eines potentiellen Integranten wurde nach der Methode von Eikmanns et al. (Microbiology 140: 1817 - 1828 (1994)) isoliert und jeweils mit den Restriktionsenzymen SalI, SacI und HinDIII geschnitten. Die entstehenden Fragmente wurden mit Agarosegel-Elektrophorese aufgetrennt und mit dem Dig-Hybrisierungskit der Firma Boehringer bei 68°C hybridisiert. Das in Beispiel 3 genannte Plasmid pCR2.1poxBint hatte innerhalb des chromosomalen poxB-Gens ins Chromosom von DSM5715 inseriert. Der Stamm wurde als DSM5715::pCR2.1poxBint bezeichnet.

### Beispiel 5

### Herstellung von Lysin

Der in Beispiel 3 erhaltene C. glutamicum Stamm DSM5715::pCR2.1poxBint wurde in einem zur Produktion von Lysin geeigneten Nährmedium kultiviert und der Lysingehalt im Kulturüberstand bestimmt.

Dazu wurde der Stamm zunächst auf Agarplatte mit dem entsprechenden Antibiotikum (Hirn-Herz Agar mit Kanamycin (25 mg/l) für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium CgIII verwendet. Diesem wurde Kanamycin (25 mg/l) zugesetzt. Die Vorkultur wurde 48 Stunden bei 33°C bei 240 rpm auf dem Schüttler inkubiert. Von dieser Vorkultur wurde eine Hauptkultur angeimpft, so daß die Anfangs-OD (660nm) der Hauptkultur 0,1 OD betrug. Für die Hauptkultur wurde das Medium MM verwendet.

| Medium MM | |
|---|---|
| CSL (Corn Steep Liquor) | 5 g/l |
| MOPS | 20 g/l |
| Glucose (getrennt autoklaviert) | 50g/l |

| Salze: | |
|---|---|
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| Leucin (sterilfiltriert) | 0,1 g/l |
| CaCO₃ | 25 g/l |

CSL, MOPS und die Salzlösung werden mit Ammoniakwasser auf pH 7 eingestellt und autoklaviert. Anschließend werden die sterilen Substrat- und Vitaminlösungen zugesetzt, sowie das trocken autoklavierte CaCO₃ zugesetzt.

Die Kultivierung erfolgt in 10 ml Volumen in einem 100 ml Erlenmeyerkolben mit Schikanen. Es wurde Kanamycin (25 mg/l) zugesetzt. Die Kultivierung erfolgte bei 33°C und 80% Luftfeuchte.

Nach 48 Stunden wurde die OD bei einer Meßwellenlänge von 660 nm mit dem Biomek 1000 (Beckmann Instruments GmbH, München) ermittelt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 1 ist das Ergebnis des Versuchs dargestellt.

**Tabelle 1**

| Stamm | OD(660) | Lysin-HCl g/l |
|---|---|---|
| DSM5715 | 13,1 | 9,5 |
| DSM5715::pCR2.1poxBint | 12,5 | 12,9 |

### Beispiel 6

### Integrationsmutagenese des poxB-Gens in dem Valinproduzenten FERM-BP 1763

Der in Beispiel 2 genannte Vektor pCR2.1poxBint wurde nach der Elektroporationsmethode von Tauch et.al. (FEMS Microbiological Letters, 123:343-347 (1994)) in Brevibacterium lactofermentum FERM-BP 1763 elektroporiert. Bei dem Stamm FERM-BP 1763 handelt es sich um einen Mycophenolsäure-resistenten Valin-Produzenten (US-A-5,188,948). Der Vektor pCR2.1poxBint kann in FERM-BP 1763 nicht selbständig replizieren und bleibt nur dann in der Zelle erhalten, wenn er ins Chromosom von FERM-BP 1763 integriert hat. Die Selektion von Klonen mit ins Chromosom integriertem pCR2.1poxBint erfolgte durch Ausplattieren des Elektroporationsansatzes auf LB Agar (Sambrook et al., Molecular cloning: a laboratory manual. 2^{nd} Ed. Cold Spring Harbor Laboratory Press, Cold Spring Harbor, N.Y.), der mit 15 mg/l Kanamycin supplementiert worden war. Für den Nachweis der Integration wurde das poxBint Fragment nach der Methode "The DIG System Users Guide for Filter Hybridization" der Firma Boehringer Mannheim GmbH (Mannheim, Deutschland, 1993) mit dem Dig-Hybridisierungskit der Firma Boehringer markiert. Chromosomale DNA eines potentiellen Integranten wurde nach der Methode von Eikmanns et al. (Microbiology 140: 1817 - 1828 (1994)) isoliert und jeweils mit den Restriktionsenzymen SalI, SacI und HinDIII geschnitten. Die entstehenden Fragmente wurden mit Agarosegel-Elektrophorese aufgetrennt und mit dem Dig-Hybrisierungskit der Firma Boehringer bei 68°C hybridisiert. Das in Beispiel 3 genannte Plasmid pCR2.1poxBint hatte innerhalb des chromosomalen poxB-Gens ins Chromosom von FERM-BP 1763 inseriert. Der Stamm wurde als FERM-BP 1763::pCR2.1poxBint bezeichnet.

### Beispiel 7

### Herstellung von Valin

Der in Beispiel 6 erhaltene B. lactofermentum Stamm FERM-BP 1763::pCR2.1poxBint wurde in einem zur Produktion von Valin geeigneten Nährmedium kultiviert und der Valingehalt im Kulturüberstand bestimmt.

Dazu wurde der Stamm zunächst auf Agarplatte mit dem entsprechenden Antibiotikum (Hirn-Herz Agar mit Kanamycin (25 mg/l) für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium CgIII verwendet. Diesem wurde Kanamycin (25 mg/l) zugesetzt. Die Vorkultur wurde 48 Stunden bei 33°C bei 240 rpm auf dem Schüttler inkubiert.

Von dieser Vorkultur wurde eine Hauptkultur angeimpft, so daß die Anfangs-OD (660nm) der Hauptkultur 0,1 OD betrug. Für die Hauptkultur wurde das Medium MM verwendet.

| Medium MM | |
|---|---|
| CSL | 5 g/l |
| MOPS | 20 g/l |
| Glucose (getrennt autoklaviert) | 50g/l |

| Salze: | |
|---|---|
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Isoleucin (sterilfiltriert) | 0,1 g/l |
| Methionin (sterilfiltriert) | 0,1 g/l |
| Thiamin * HCl (sterilfiltriert) | 0,2 mg/l |
| Leucin (sterilfiltriert) | 0,1 g/l |
| CaCO₃ | 25 g/l |

CSL (Corn Steep Liquor), MOPS (Morpholinopropansulfonsäure) und die Salzlösung werden mit Ammoniakwasser auf pH 7 eingestellt und autoklaviert. Anschließend werden die sterilen Substrat- und Vitaminlösungen zugesetzt, sowie das trocken autoklavierte CaCO₃ zugesetzt.

Die Kultivierung erfolgt in 10 ml Volumen in einem 100 ml Erlenmeyerkolben mit Schikanen. Es wurde Kanamycin (25 mg/l) zugesetzt. Die Kultivierung erfolgte bei 33°C und 80% Luftfeuchte.

Nach 48 Stunden wurde die OD bei einer Meßwellenlänge von 660 nm mit dem Biomek 1000 (Beckmann Instruments GmbH, München) ermittelt. Die gebildete Valinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 2 ist das Ergebnis des Versuchs dargestellt.

**Tabelle 2**

| Stamm | OD(660) | Valin-HCl g/l |
|---|---|---|
| FERM-BP 1763 | 8,6 | 12,1 |
| FERM-BP 1763::pCR2.1poxBint | 9,5 | 13,0 |

Folgende Figuren sind beigefügt:
- Figur 1:: Karte des Plasmids pCR2.1poxBint.

Die verwendeten Abkürzungen und Bezeichnungen haben folgende Bedeutung.
- ColE1 ori:: Replikationsursprung des Plasmids ColE1
- lacZ:: 5*'*Ende des β-Galactosidase Gens
- f1 ori:: Replikationsursprung des Phagen f1
- KmR:: Kanamycin Resistenz
- ApR:: Ampicillin Resistenz
- BamHI:: Schnittstelle des Restriktionsenzyms BamHI
- EcoRI:: Schnittstelle des Restriktionsenzyms EcoRI
- poxBint:: internes Fragment des poxB-Gens

## Patentansprüche

1. Isoliertes Polynukleotid enthaltend eine Polynukleotidsequenz, ausgewählt aus der Gruppe
a) Polynukleotid, das zu mindestens 70 % identisch ist mit einem Polynukleotid, das für ein Polypeptid codiert, das die Aminosäuresequenz von SEQ ID No. 2 enthält,
b) Polynukleotid, das für ein Polypeptid codiert, das eine Aminosäuresequenz enthält, die zu mindestens 70 % identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Basen der Polynukleotidsequenz von a), b) oder c).

2. Polynukleotid gemäß Anspruch 1,
wobei das Polynukleotid eine replizierbare, bevorzugt rekombinante DNA ist.

3. Polynukleotid gemäß Anspruch 1, wobei das Polynukleotid eine RNA ist.

4. Polynukleotid gemäß Anspruch 2,
enthaltend die Nukleinsäuresequenz wie in SEQ ID No. 1 dargestellt.

5. Polynukleotidsequenz gemäß Anspruch 2, die für ein Polypeptid codiert, das die Aminosäuresequenz in SEQ ID No. 2 darstellt, enthält.

6. Replizierbare DNA gemäß Anspruch 2,
enthaltend
(i) die Nukleotidsequenz, gezeigt in SEQ-ID-No. 1, oder
(ii) mindestens eine Sequenz, die der Sequenz (i) innerhalb des Bereichs der Degeneration des genetischen Codes einspricht, oder
(iii) mindestens eine Sequenz, die mit der zur Sequenz (i) oder (ii) komplementären Sequenz hybridisiert, und gegebenenfalls
(iv) funktionsneutrale Sinnmutanten in (i)

7. Vektor, enthaltend das Polynukleotid gemäß Anspruch 1, insbesondere Punkt d, hinterlegt in E.coli, DSM 13114.

8. Als Wirtszelle dienende coryneforme Bakterien, die eine Deletion oder eine Insertion in dem poxB-Gen enthalten.

9. Verfahren zur Herstellung von L-Aminosäuren, insbesondere L-Lysin,
**dadurch gekennzeichnet**,
daß man folgende Schritte durchführt,
a) Fermentation der die gewünschte L-Aminosäure produzierenden Bakterien, in denen man zumindest das poxB-Gen abschwächt,
b) Anreicherung des gewünschten L-Aminosäure im Medium oder in den Zellen der Bakterien, und
c) Isolieren der L-Aminosäure.

10. Verfahren gemäß Anspruch 9,
**dadurch gekennzeichnet**,
daß man Bakterien einsetzt, in denen man zusätzlich weitere Gene des Biosyntheseweges der gewünschten L-Aminosäure verstärkt.

11. Verfahren gemäß Anspruch 9,
**dadurch gekennzeichnet**,
daß man Bakterien einsetzt, in denen die Stoffwechselwege zumindest teilweise ausgeschaltet sind, die die Bildung der gewünschten L-Aminosäure verringern.

12. Verfahren gemäß Anspruch 9,
**dadurch gekennzeichnet**,
daß man die Expression des Polynukleotids gemäß Anspruch 1, insbesondere 1 a bis 1 c verringert.

13. Verfahren gemäß Anspruch 9,
**dadurch gekennzeichnet**,
daß man die katalytischen Eigenschaften des Polypeptids (Enzymproteins) herabsetzt, für das das Polynukleotid gemäss Anspruch 1, insbesondere 1 a bis 1 c codiert.

14. Verfahren gemäß Anspruch 9,
**dadurch gekennzeichnet**,
daß man Bakterien einsetzt, in denen man zur Abschwächung die Integrationsmutagenese mittels des Plasmids pCR2.1poxBint, dargestellt in Figur 1 und hinterlegt als DSM 13114, oder eines seiner Bestandteile verwendet.

15. Verfahren gemäß Anspruch 9,
**dadurch gekennzeichnet**,
daß man für die Herstellung von L-Lysin Bakterien fermentiert, in denen man gleichzeitig eines oder mehrere Gene überexprimiert, ausgewählt aus der Gruppe
• das für die Dihydrodipicolinat-Synthase kodierende dapA-Gen,
• das die S-(2-Aminoethyl)-Cystein-Resistenz vermittelnde DNA-Fragment,
• das die Pyruvat-Carboxylase kodierende pyc-Gen,
• das Gen für die Succinyldiaminopimelate-Desuccinylase kodierende dapE Gen
• das für die Glyceraldehyd-3-Phosphat Dehydrogenase kodierende dap-Gen
• das für die Malat:Chinon Oxidoreduktase kodierende mqo-Gen
• das für den Lysin-Export kodierende lysE-Gen.

16. Verfahren gemäß einem oder mehreren der vorhergehenden Ansprüche,
**dadurch gekennzeichnet**,
daß man Mikroorganismen der Gattung Corynebacterium glutamicum einsetzt.


SEQUENZPROTOKOLL

<110> Degussa-Hüls AG

<120> Neue für das poxB-Gen codierende Nukleotidsequenzen

<130> 990159 BT

<140>
<141>

<160> 3

<170> PatentIn Ver. 2.1

<210> 1
<211> 2160
<212> DNA
<213> Corynebacterium glutamicum

<220>
<221> CDS
<222> (327)..(2063)

<220>
<221> -35_signal
<222> (227)..(232)

<220>
<221> -10_signal
<222> (256)..(261)

<400> 1
ttagaggcga ttctgtgagg tcactttttg tggggtcggg gtctaaattt ggccagtttt 60

cgaggcgacc agacaggcgt gcccacgatg tttaaatagg cgatcggtgg gcatctgtgt 120

ttggtttcga cgggctgaaa ccaaaccaga ctgcccagca acgacggaaa tcccaaaagt 180

gggcatccct gtttggtacc gagtacccac ccgggcctga aactccctgg caggcgggcg 240

aagcgtggca acaactggaa tttaagagca caattgaagt cgcaccaagt taggcaacac 300

aatagccata acgttgagga gttcag atg gca cac agc tac gca gaa caa tta 353
                             Met Ala His Ser Tyr Ala Glu Gln Leu
                              1               5

att gac act ttg gaa gct caa ggt gtg aag cga att tat ggt ttg gtg 401
Ile Asp Thr Leu Glu Ala Gln Gly Val Lys Arg Ile Tyr Gly Leu Val
 10                  15                  20                  25

ggt gac agc ctt aat ccg atc gtg gat gct gtc cgc caa tca gat att 449
Gly Asp Ser Leu Asn Pro Ile Val Asp Ala Val Arg Gln Ser Asp Ile
                 30                  35                  40

gag tgg gtg cac gtt cga aat gag gaa gcg gcg gcg ttt gca gcc ggt 497
Glu Trp Val His Val Arg Asn Glu Glu Ala Ala Ala Phe Ala Ala Gly
             45                  50                  55

gcg gaa tcg ttg atc act ggg gag ctg gca gta tgt gct gct tct tgt    545
Ala Glu Ser Leu Ile Thr Gly Glu Leu Ala Val Cys Ala Ala Ser Cys
            60                  65                  70

ggt cct gga aac aca cac ctg att cag ggt ctt tat gat tcg cat cga    593
Gly Pro Gly Asn Thr His Leu Ile Gln Gly Leu Tyr Asp Ser His Arg
        75                  80                  85

aat ggt gcg aag gtg ttg gcc atc gct agc cat att ccg agt gcc cag    641
Asn Gly Ala Lys Val Leu Ala Ile Ala Ser His Ile Pro Ser Ala Gln
 90                  95                 100                 105

att ggt tcg acg ttc ttc cag gaa acg cat ccg gag att ttg ttt aag    689
Ile Gly Ser Thr Phe Phe Gln Glu Thr His Pro Glu Ile Leu Phe Lys
                110                 115                 120

gaa tgc tct ggt tac tgc gag atg gtg aat ggt ggt gag cag ggt gaa    737
Glu Cys Ser Gly Tyr Cys Glu Met Val Asn Gly Gly Glu Gln Gly Glu
            125                 130                 135

cgc att ttg cat cac gcg att cag tcc acc atg gcg ggt aaa ggt gtg    785
Arg Ile Leu His His Ala Ile Gln Ser Thr Met Ala Gly Lys Gly Val
            140                 145                 150

tcg gtg gta gtg att cct ggt gat atc gct aag gaa gac gca ggt gac    833
Ser Val Val Val Ile Pro Gly Asp Ile Ala Lys Glu Asp Ala Gly Asp
        155                 160                 165

ggt act tat tcc aat tcc act att tct tct ggc act cct gtg gtg ttc    881
Gly Thr Tyr Ser Asn Ser Thr Ile Ser Ser Gly Thr Pro Val Val Phe
170                 175                 180                 185

ccg gat cct act gag gct gca gcg ctg gtg gag gcg att aac aac gct    929
Pro Asp Pro Thr Glu Ala Ala Ala Leu Val Glu Ala Ile Asn Asn Ala
                190                 195                 200

aag tct gtc act ttg ttc tgc ggt gcg ggc gtg aag aat gct cgc gcg    977
Lys Ser Val Thr Leu Phe Cys Gly Ala Gly Val Lys Asn Ala Arg Ala
            205                 210                 215

cag gtg ttg gag ttg gcg gag aag att aaa tca ccg atc ggg cat gcg    1025
Gln Val Leu Glu Leu Ala Glu Lys Ile Lys Ser Pro Ile Gly His Ala
        220                 225                 230

ctg ggt ggt aag cag tac atc cag cat gag aat ccg ttt gag gtc ggc    1073
Leu Gly Gly Lys Gln Tyr Ile Gln His Glu Asn Pro Phe Glu Val Gly
    235                 240                 245

atg tct ggc ctg ctt ggt tac ggc gcc tgc gtg gat gcg tcc aat gag    1121
Met Ser Gly Leu Leu Gly Tyr Gly Ala Cys Val Asp Ala Ser Asn Glu
250                 255                 260                 265

gcg gat ctg ctg att cta ttg ggt acg gat ttc cct tat tct gat ttc    1169
Ala Asp Leu Leu Ile Leu Leu Gly Thr Asp Phe Pro Tyr Ser Asp Phe
                270                 275                 280

ctt cct aaa gac aac gtt gcc cag gtg gat atc aac ggt gcg cac att    1217
Leu Pro Lys Asp Asn Val Ala Gln Val Asp Ile Asn Gly Ala His Ile
            285                 290                 295

ggt cga cgt acc acg gtg aag tat ccg gtg acc ggt gat gtt gct gca 1265
Gly Arg Arg Thr Thr Val Lys Tyr Pro Val Thr Gly Asp Val Ala Ala
        300                 305                 310

aca atc gaa aat att ttg cct cat gtg aag gaa aaa aca gat cgt tcc 1313
Thr Ile Glu Asn Ile Leu Pro His Val Lys Glu Lys Thr Asp Arg Ser
    315                 320                 325

ttc ctt gat cgg atg ctc aag gca cac gag cgt aag ttg agc tcg gtg 1361
Phe Leu Asp Arg Met Leu Lys Ala His Glu Arg Lys Leu Ser Ser Val
330                 335                 340                 345

gta gag acg tac aca cat aac gtc gag aag cat gtg cct att cac cct 1409
Val Glu Thr Tyr Thr His Asn Val Glu Lys His Val Pro Ile His Pro
                350                 355                 360

gaa tac gtt gcc tct att ttg aac gag ctg gcg gat aag gat gcg gtg 1457
Glu Tyr Val Ala Ser Ile Leu Asn Glu Leu Ala Asp Lys Asp Ala Val
            365                 370                 375

ttt act gtg gat acc ggc atg tgc aat gtg tgg cat gcg agg tac atc 1505
Phe Thr Val Asp Thr Gly Met Cys Asn Val Trp His Ala Arg Tyr Ile
        380                 385                 390

gag aat ccg gag gga acg cgc gac ttt gtg ggt tca ttc cgc cac ggc 1553
Glu Asn Pro Glu Gly Thr Arg Asp Phe Val Gly Ser Phe Arg His Gly
    395                 400                 405

acg atg gct aat gcg ttg cct cat gcg att ggt gcg caa agt gtt gat 1601
Thr Met Ala Asn Ala Leu Pro His Ala Ile Gly Ala Gln Ser Val Asp
410                 415                 420                 425

cga aac cgc cag gtg atc gcg atg tgt ggc gat ggt ggt ttg ggc atg 1649
Arg Asn Arg Gln Val Ile Ala Met Cys Gly Asp Gly Gly Leu Gly Met
                430                 435                 440

ctg ctg ggt gag ctt ctg acc gtt aag ctg cac caa ctt ccg ctg aag 1697
Leu Leu Gly Glu Leu Leu Thr Val Lys Leu His Gln Leu Pro Leu Lys
            445                 450                 455

gct gtg gtg ttt aac aac agt tct ttg ggc atg gtg aag ttg gag atg 1745
Ala Val Val Phe Asn Asn Ser Ser Leu Gly Met Val Lys Leu Glu Met
        460                 465                 470

ctc gtg gag gga cag cca gaa ttt ggt act gac cat gag gaa gtg aat 1793
Leu Val Glu Gly Gln Pro Glu Phe Gly Thr Asp His Glu Glu Val Asn
    475                 480                 485

ttc gca gag att gcg gcg gct gcg ggt atc aaa tcg gta cgc atc acc 1841
Phe Ala Glu Ile Ala Ala Ala Ala Gly Ile Lys Ser Val Arg Ile Thr
490                 495                 500                 505

gat ccg aag aaa gtt cgc gag cag cta gct gag gca ttg gca tat cct 1889
Asp Pro Lys Lys Val Arg Glu Gln Leu Ala Glu Ala Leu Ala Tyr Pro
                510                 515                 520

gga cct gta ctg atc gat atc gtc acg gat cct aat gcg ctg tcg atc 1937
Gly Pro Val Leu Ile Asp Ile Val Thr Asp Pro Asn Ala Leu Ser Ile
            525                 530                 535

cca cca acc atc acg tgg gaa cag gtc atg gga ttc agc aag gcg gcc 1985
Pro Pro Thr Ile Thr Trp Glu Gln Val Met Gly Phe Ser Lys Ala Ala
        540                 545                 550

acc cga acc gtc ttt ggt gga gga gta gga gcg atg atc gat ctg gcc 2033
Thr Arg Thr Val Phe Gly Gly Gly Val Gly Ala Met Ile Asp Leu Ala
    555                 560                 565

cgt tcg aac ata agg aat att cct act cca tgatgattga tacacctgct 2083
Arg Ser Asn Ile Arg Asn Ile Pro Thr Pro
570                 575

gttctcattg accgcgagcg cttaactgcc aacatttcca ggatggcagc tcacgccggt 2143

gcccatgaga ttgccct 2160

<210> 2
<211> 579
<212> PRT
<213> Corynebacterium glutamicum

<400> 2
Met Ala His Ser Tyr Ala Glu Gln Leu Ile Asp Thr Leu Glu Ala Gln
1               5                   10                  15

Gly Val Lys Arg Ile Tyr Gly Leu Val Gly Asp Ser Leu Asn Pro Ile
            20                  25                  30

Val Asp Ala Val Arg Gln Ser Asp Ile Glu Trp Val His Val Arg Asn
        35                  40                  45

Glu Glu Ala Ala Ala Phe Ala Ala Gly Ala Glu Ser Leu Ile Thr Gly
    50                  55                  60

Glu Leu Ala Val Cys Ala Ala Ser Cys Gly Pro Gly Asn Thr His Leu
65                  70                  75                  80

Ile Gln Gly Leu Tyr Asp Ser His Arg Asn Gly Ala Lys Val Leu Ala
                85                  90                  95

Ile Ala Ser His Ile Pro Ser Ala Gln Ile Gly Ser Thr Phe Phe Gln
            100                 105                 110

Glu Thr His Pro Glu Ile Leu Phe Lys Glu Cys Ser Gly Tyr Cys Glu
        115                 120                 125

Met Val Asn Gly Gly Glu Gln Gly Glu Arg Ile Leu His His Ala Ile
    130                 135                 140

Gln Ser Thr Met Ala Gly Lys Gly Val Ser Val Val Val Ile Pro Gly
145                 150                 155                 160

Asp Ile Ala Lys Glu Asp Ala Gly Asp Gly Thr Tyr Ser Asn Ser Thr
                165                 170                 175

Ile Ser Ser Gly Thr Pro Val Val Phe Pro Asp Pro Thr Glu Ala Ala
            180                 185                 190

Ala Leu Val Glu Ala Ile Asn Asn Ala Lys Ser Val Thr Leu Phe Cys
        195                 200                 205

Gly Ala Gly Val Lys Asn Ala Arg Ala Gln Val Leu Glu Leu Ala Glu
    210                 215                 220

Lys Ile Lys Ser Pro Ile Gly His Ala Leu Gly Gly Lys Gln Tyr Ile
225                 230                 235                 240

Gln His Glu Asn Pro Phe Glu Val Gly Met Ser Gly Leu Leu Gly Tyr
                245                 250                 255

Gly Ala Cys Val Asp Ala Ser Asn Glu Ala Asp Leu Leu Ile Leu Leu
            260                 265                 270

Gly Thr Asp Phe Pro Tyr Ser Asp Phe Leu Pro Lys Asp Asn Val Ala
        275                 280                 285

Gln Val Asp Ile Asn Gly Ala His Ile Gly Arg Arg Thr Thr Val Lys
    290                 295                 300

Tyr Pro Val Thr Gly Asp Val Ala Ala Thr Ile Glu Asn Ile Leu Pro
305                 310                 315                 320

His Val Lys Glu Lys Thr Asp Arg Ser Phe Leu Asp Arg Met Leu Lys
                325                 330                 335

Ala His Glu Arg Lys Leu Ser Ser Val Val Glu Thr Tyr Thr His Asn
            340                 345                 350

Val Glu Lys His Val Pro Ile His Pro Glu Tyr Val Ala Ser Ile Leu
        355                 360                 365

Asn Glu Leu Ala Asp Lys Asp Ala Val Phe Thr Val Asp Thr Gly Met
    370                 375                 380

Cys Asn Val Trp His Ala Arg Tyr Ile Glu Asn Pro Glu Gly Thr Arg
385                 390                 395                 400

Asp Phe Val Gly Ser Phe Arg His Gly Thr Met Ala Asn Ala Leu Pro
                405                 410                 415

His Ala Ile Gly Ala Gln Ser Val Asp Arg Asn Arg Gln Val Ile Ala
            420                 425                 430

Met Cys Gly Asp Gly Gly Leu Gly Met Leu Leu Gly Glu Leu Leu Thr
        435                 440                 445

Val Lys Leu His Gln Leu Pro Leu Lys Ala Val Val Phe Asn Asn Ser
    450                 455                 460

Ser Leu Gly Met Val Lys Leu Glu Met Leu Val Glu Gly Gln Pro Glu
465                 470                 475                 480

Phe Gly Thr Asp His Glu Glu Val Asn Phe Ala Glu Ile Ala Ala Ala
                485                 490                 495

Ala Gly Ile Lys Ser Val Arg Ile Thr Asp Pro Lys Lys Val Arg Glu
            500                 505                 510

Gln Leu Ala Glu Ala Leu Ala Tyr Pro Gly Pro Val Leu Ile Asp Ile
        515                 520                 525

Val Thr Asp Pro Asn Ala Leu Ser Ile Pro Pro Thr Ile Thr Trp Glu
    530                 535                 540

Gln Val Met Gly Phe Ser Lys Ala Ala Thr Arg Thr Val Phe Gly Gly
545                 550                 555                 560

Gly Val Gly Ala Met Ile Asp Leu Ala Arg Ser Asn Ile Arg Asn Ile
                565                 570                 575

Pro Thr Pro

<210> 3
<211> 875
<212> DNA
<213> Corynebacterium glutamicum

<400> 3
tgcgagatgg tgaatggtgg tgagcagggt gaacgcattt tgcatcacgc gattcagtcc 60
accatggcgg gtaaaggtgt gtcggtggta gtgattcctg gtgatatcgc taaggaagac 120
gcaggtgacg gtacttattc caattccact atttcttctg gcactcctgt ggtgttcccg 180
gatcctactg aggctgcagc gctggtggag gcgattaaca acgctaagtc tgtcactttg 240
ttctgcggtg cgggcgtgaa gaatgctcgc gcgcaggtgt tggagttggc ggagaagatt 300
aaatcaccga tcgggcatgc gctgggtggt aagcagtaca tccagcatga gaatccgttt 360
gaggtcggca tgtctggcct gcttggttac ggcgcctgcg tggatgcgtc caatgaggcg 420
gatctgctga ttctattggg tacggatttc ccttattctg atttccttcc taaagacaac 480
gttgcccagg tggatatcaa cggtgcgcac attggtcgac gtaccacggt gaagtatccg 540
gtgaccggtg atgttgctgc aacaatcgaa aatattttgc ctcatgtgaa ggaaaaaaca 600
gatcgttcct tccttgatcg gatgctcaag gcacacgagc gtaagttgag ctcggtggta 660
gagacgtaca cacataacgt cgagaagcat gtgcctattc accctgaata cgttgcctct 720
attttgaacg agctggcgga taaggatgcg gtgtttactg tggataccgg catgtgcaat 780
gtgtggcatg cgaggtacat cgagaatccg gagggaacgc gcgactttgt gggttcattc 840
cgccacggca cgatggctaa tgcgttgcct catgc 875